# EUROPEAN PATENT APPLICATION

(11) **EP 4 223 885 A1**
(43) Date of publication of application: **09.08.2023**
(21) Application number: 21875541.1
(22) Date of filing: 27.09.2021
(51) Int. Cl.: C12Q 1/02, G01N 33/15, G01N 33/50

(54) **METHOD FOR EVALUATING PLATE AND METHOD FOR EVALUATING TOXICITY OF SPECIMEN**

(30) Priority: 29.09.2020 JP 2020164030
(71) Applicant: SUMITOMO CHEMICAL COMPANY, LIMITED, Tokyo 103-6020 (JP)
(72) Inventor: YAMADA, Sachiko, Osaka-shi, Osaka 554-8558 (JP); KANO, Hanaho, Osaka-shi, Osaka 554-8558 (JP); OZAKI, Maya, Niihama-shi, Ehime 792-8521 (JP)
(74) Representative: Vossius & Partner Patentanwälte Rechtsanwälte mbB
(86) International application number: PCT/JP2021/035434
(87) International publication number: WO 2022/071239

(57) **Abstract**

A method for evaluating a plate according to an embodiment is a method for evaluating a plate including a plurality of wells, the method including: an injection step of injecting an evaluation sample including a cell and a test substance into the plurality of wells, the evaluation sample being adjusted in advance so that a foreign matter is generated as a result of cell death caused by the test substance; a counting step of counting the number of wells having the foreign matter among the plurality of wells at a first counting timing at which a first predetermined time has elapsed since the evaluation sample is injected into the plurality of wells; and a determination step of determining that the plate is appropriate when the number of wells having the foreign matter among the plurality of wells is one or more, and determining that the plate is inappropriate when the number of wells having the foreign matter is zero.

## Description

### TECHNICAL FIELD

The present invention relates to a method for evaluating a plate and a method for evaluating toxicity of a test substance.

### BACKGROUND ART

In biological safety evaluation, it is known to add a test substance to a tester and observe the change of the tester. For example, in the Ames test, cells which are improved not so as to synthesize amino acid by performing genetic manipulation on an amino acid synthesis gene are used as a tester. In the Ames test, a test substance is added to the cells, and then the cells are cultured under certain conditions. When mutation occurs in the amino acid synthesis gene of the cells by the test substance, the cells can synthesize amino acid again. Therefore, whether or not mutation has occurred is evaluated by confirming the presence or absence of cell proliferation. At this time, for example, when an indicator that changes in color according to the proliferation of cells is used, the proliferation of cells can be evaluated by the change in color. When cell death occures due to the influence of the test substance, a foreign matter may be generated. Therefore, the toxicity of the test substance to the cells can be evaluated by confirming the presence or absence of a foreign matter.

In recent years, the Ames test using a plate having a plurality of wells as disclosed in, for example, Non-Patent Documents 1, 2, and 3 is known in order to reduce the amounts of tester and test substance, or the like.

### PRIOR ART DOCUMENTS

### NON-PATENT DOCUMENTS

Non-patent Document 1: Fluckiger-Isler S., Kamber M., "Direct comparison of the Ames microplate format(MPF) test in liquid medium with the standard Ames pre-incubation assay on agar plates by use of equivocal to weakly positive test compounds.", Mutation Research/Genetic Toxicology and Environmental Mutagenesis 747, p36-45, 2012.

Non-patent Document 2: Sui H., Kawakami K., Sakurai N., Hara T., Nohmi T., "Improvement and evaluation of high throughput fluctuation Ames test using 384-well plate with Salmonella typhimurium TA100 and TA98.", Genes and Environment 31(2), p47-55, 2009.

Non-patent Document 3: Kamber M., Fluckiger-Isler S., Engelhardt G., Jaeckh R., Zeiger E., "Comparison of the Ames II and traditional Ames test responses with respect to mutagenicity, strain specificities, need for metabolism and correlation with rodent carcinogenicity", Mutagenesis 24(4),p359-366, 2009.

### SUMMARY OF THE INVENTION

### PROBLEMS TO BE SOLVED BY THE INVENTION

When cytotoxicity is evaluated using a plate having a plurality of wells as disclosed in Non-Patent Documents 1, 2, and 3, for example, even when testers and test substances under the same conditions are used, the evaluation may vary depending on the plate. For example, even under a condition where cell death occures due to the influence of the test substance and a foreign matter is generated, the foreign matter may disappear due to the plate. In this case, the safety evaluation cannot be properly performed.

Therefore, an object of the present invention is to provide a method for evaluating a plate, the method being capable of evaluating the appropriateness of a plate used in biological safety evaluation, and a method for evaluating toxicity of a test substance using the plate evaluated by the evaluation method.

### MEANS FOR SOLVING THE PROBLEMS

A method for evaluating a plate according to one aspect of the present invention is a method for evaluating a plate including a plurality of wells, the method including: an injection step of injecting an evaluation sample including a cell and a test substance into the plurality of wells, the evaluation sample being adjusted in advance so that a foreign matter is generated as a result of cell death caused by the test substance; a counting step of counting the number of wells having the foreign matter among the plurality of wells at a first counting timing at which a first predetermined time has elapsed since the evaluation sample is injected into the plurality of wells; and a determination step of determining that the plate is appropriate when the number of wells having the foreign matter among the plurality of wells is one or more, and determining that the plate is inappropriate when the number of wells having the foreign matter is zero.

In the method for evaluating a plate, whether or not a foreign matter is present in the well is counted at the first counting timing at which the first predetermined time has elapsed since the evaluation sample is injected into the plurality of wells. When the first predetermined time elapses after the evaluation sample is injected into the well, the reliable presence of the foreign matter in the well can be ensured in a case where the plate is a plate that can retain the foreign matter. Therefore, by counting whether or not a foreign matter is present in the well at the first counting timing as described above, it is possible to evaluate whether or not the plate can retain a foreign matter, that is, whether or not the plate is suitable for the biological safety evaluation test in the method for evaluating a plate.

The first counting timing may be after the evaluation sample is cultured for 48 hours from injection of the evaluation sample into the plurality of wells. In this case, when the number of wells including the foreign matter is present is counted, it is easy to ensure a state in which the foreign matter is present in the well.

The plurality of wells may have 48 or more wells.

The determination step may be performed again after the number of wells having a foreign matter among the plurality of wells is counted at a second counting timing at which a second predetermined time has elapsed while the plate is stored at a constant temperature from the first counting timing.

Even in a biological safety evaluation test in which the safety is not evaluated at the first counting timing, but is evaluated after the plate is stored at a constant temperature for a predetermined time, it is possible to evaluate whether or not the plate is an appropriate plate by the evaluation method.

The constant temperature may be 10°C or lower.

The cell may be a bacterium. The cell may be *E. coli* or *S. typhimurium.*

The evaluation sample may be a sample in which a cell and a test substance are adjusted so that at least one well has the foreign matter when the number of wells having the foreign matter among a plurality of wells of a foreign matter retaining plate is counted at the first counting timing at which the first predetermined time has elapsed since the evaluation sample is injected into the plurality of wells of the foreign matter retaining plate, and the foreign matter retaining plate may be a plate including: a glass plate; and a resin layer that is disposed on a surface of the glass plate and is formed of a cyclic olefin copolymer, wherein a plurality of through holes to be the plurality of wells of the foreign matter retaining plate are formed in the resin layer, and a region exposed through the plurality of through holes in the surface is coated with fibronectin.

The test substance may be tert-butylhydroquinone, 1-decanol, 2-ethoxynaphthalene, 4-(1,1,3,3-tetramethylbutyl)phenol, diethyl chlorothiophosphate, or 4-chlorobenzyl chloride. The test substance may be 1-decanol having a concentration of 62.5 µg/mL or more, or tert-butylhydroquinone having a concentration of 667 ug/mL or more.

The evaluation sample may be, for example, an evaluation sample using an *E. coli* WP2 uvrA strain as the cell and 1-decanol as the test substance having a concentration of 62.5 µg/mL or 125 µg/mLtest substance, or an evaluation sample using a *S. typhimurium* TA100 strain as the cell and 1-decanol as the test substance having a concentration of 667 µg/mL or 2,000 µg/mLtest substance.

The method for evaluating toxicity of a test substance according to one aspect of the present invention includes a step of injecting an evaluation target including: a tester including a cell; and a test substance into a plurality of wells of the plate determined to be appropriate by the method for evaluating a plate described above, and evaluating toxicity of the test substance. In this case, the toxicity of the test substance can be accurately evaluated.

### EFFECT OF THE INVENTION

According to the present invention, it is possible to provide a method for evaluating a plate, the method being capable of evaluating the appropriateness of a plate used in biological safety evaluation, and a method for evaluating toxicity of a test substance using the plate evaluated by the evaluation method.

### BRIEF DESCRIPTION OF THE DRAWINGS

Fig. 1 is a plan view of an example of a plate.
Fig. 2 is a schematic view of a cross section of a well.
Fig. 3 is a flowchart of a method for evaluating a plate according to an embodiment.

### MODE FOR CARRYING OUT THE INVENTION

Hereinafter, embodiments of the present invention will be described with reference to the drawings. The same elements are denoted by the same reference numerals, and redundant description will be omitted. The dimensional ratios in the drawings do not necessarily coincide with those in the description.

In the present embodiment, a method for evaluating the appropriateness of a plate used for toxicity evaluation (safety evaluation) of a test substance using the Ames test will be described.

The outline of the Ames test used in the present embodiment will be described. In the Ames test, cells which are improved not so as to synthesize amino acid by performing genetic manipulation on an amino acid synthesis gene are used as a tester. In the Ames test, a test substance is added to the cells. Thereafter, the cells are cultured under certain conditions. When mutation occurs in the amino acid synthesis gene of the cells by the test substance, the cells can synthesize amino acid again. Therefore, whether or not mutation has occurred is evaluated by confirming the presence or absence of cell proliferation, thereby evaluating genotoxicity. In addition, when cell death occures due to the influence of the test substance, a foreign matter is generated. Therefore, the method of the Ames test also functions as a cytotoxicity evaluation test for confirming whether or not the test substance has toxicity to the cells. In the following description of embodiments based on the Ames test, unless indicated otherwise, the tester is a cell.

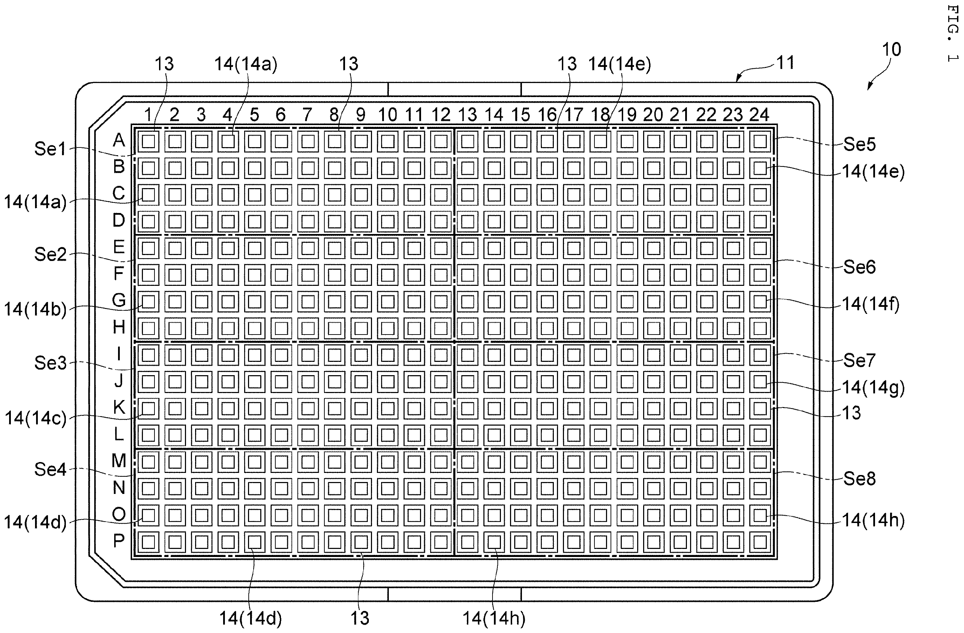

Fig. 1 is a plan view of an evaluation plate 10 used in an evaluation method according to an embodiment. The evaluation plate 10 includes a plate 11. The plate 11 has a plurality of wells 13. In the present embodiment, the plate 11 has 384 wells 13 arranged two-dimensionally (16 × 24). The plate 11 in the present embodiment is a plate that has been evaluated as being appropriate by a method for evaluating the plate 11, described later. The plate 11 is, for example, a polystyrene plate to which coating is not applied. The Ames test using the plate 11 having a plurality of wells 13 as described above is also referred to as a miniaturized Ames test.

In the plate 11, the first to N-th sections (N is an integer of 2 or more) are virtually set. In the present embodiment, as illustrated in Fig. 1, a case where N is 8, that is, a case where eight sections are virtually set will be described. The eight sections are hereinafter referred to as sections Se1 to Se8. The sections Se1 to Se8 are regions each including 4 × 12 (48) wells 13 (well group). The section Se1 is a solvent control section and the section Se8 is a positive control section. The solvent control section serves as a negative control section.

The well 13 is a recess (depression) formed in the plate 11. The well 13 holds an evaluation target 14. In a case where the evaluation targets 14 held in the wells 13 belonging to the respective sections Se1 to Se8 are distinguished and described, they are referred to as evaluation targets 14a to 14h.

The evaluation targets 14a to 14h each have a culture solution in which cells are dispersed (or suspended). The same amount of culture solution is held in all 384 wells 13. The amount of cells in the culture solution held in the well 13 is also the same among the wells 13. Examples of the cell include microorganisms having amino acid auxotrophic mutation and cells derived from organisms. Microorganisms having amino acid auxotrophic mutation are histidine auxotrophic *S. typhimurium* (for example, TA1535, TA1537, TA97, TA97a, TA98, TA100, TA92, TA94, TA102, TA104, TA1538, TA7001, TA7002, TA7003, TA7004, TA7005, TA7006, and various YG strains), tryptophan auxotrophic *E. coli* (for example, WP2, WP2 uvrA, WP2/pKM 101, and WP2 uvrA/pKM 101), other microorganisms, and the like. Examples of the cell derived from organisms include cultured cells derived from mammals, cells derived from other vertebrates, and cells derived from insects. The cells included in the evaluation targets 14a to 14h may be cells in which a plurality of types of cells are mixed. An example of the culture solution is a phosphate buffer containing a trace amount of amino acid.

The evaluation targets 14a to 14h further include an indicator. The amount of the indicator is the same in the evaluation targets 14a to 14h (i.e., the same in all wells 13). In the present embodiment, the indicator is a pH indicator. An example of the pH indicator is Bromocresol Purple. In the present embodiment, the color of the pH indicator is purple in the case of a negative result (in the case where no mutation has occurred in the cell), and is yellow in the case of a positive result (in the case where mutation has occurred in the cell).

The evaluation targets 14b to 14g further include a liquid (test substance solution) in which the test substance is dissolved or uniformly dispersed in a solvent, in addition to the culture solution in which the cells are dispersed and the indicator. Therefore, the wells 13 of the sections Se2 to Se7 that hold the evaluation targets 14b to 14g are wells (test substance wells) that hold the test substance. The test substance is a substance (for example, a chemical substance) whose safety (genotoxicity and toxicity to cells in the present embodiment) is to be evaluated. Examples of the solvent include water and dimethylsulfoxide (DMSO). The evaluation targets 14b to 14g are the same except that the concentration of the test substance is different. The amounts of the test substance solutions in the evaluation targets 14b of all the wells 13 belonging to the section Se2 are the same. The same applies to the sections Se3 to Se7.

The evaluation target 14a includes a solvent control solution in addition to the culture solution containing the cells and the indicator. Therefore, the wells 13 of the section Se1 holding the evaluation targets 14a are solvent control wells. The solvent control solution is a solvent used for the test substance solution. However, the solvent control solution may be any liquid that does not affect cells. Examples of the solvent control solution include water and DMSO. The amounts of the solvent control solutions in the evaluation targets 14a are the same in all the wells 13 belonging to the section Se1.

The evaluation target 14h includes a positive control solution in addition to the culture solution containing the cells and the indicator. Therefore, the wells 13 of the section Se8 holding the evaluation targets 14h are positive control wells. The positive control solution is a solution containing a positive control compound and a solvent. The positive control compound may be any compound known to be positive for cells. Examples of the positive control compound include 9AA (9-aminoacridine), 4-NQO (4-nitroquinoline-N-oxide), 2-AA (2-aminoanthracene), and 2-NF (2-nitrofluorene). The solvent need not be the same as the solvent used for the test substance solution. Examples of the solvent include water and DMSO. The amounts of the positive control solutions held in all wells 13 belonging to the section Se8 as a positive section are the same.

When the influence of the presence or absence of metabolic activation of the test substance is examined, the evaluation targets 14a to 14h further include S9 mix. S9mix is a liquid obtained by adding a coenzyme to an animal liver extract.

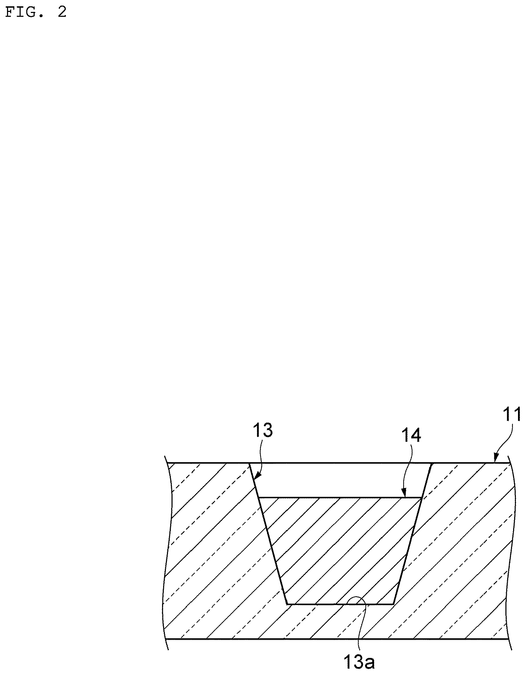

The configuration of the well 13 will be further described with reference to Figs. 1 and 2. Fig. 2 is a schematic view of a cross section of a well. Fig. 2 schematically illustrates the evaluation target 14. In the present embodiment, the plan view shape of the well 13 (the shape viewed from the depth direction of the well 13) is a square. An example of the length of one side of the opening of the well 13 is 3.0 mm to 4.0 mm. An example of the depth of the well 13 is 9.0 mm to 15.0 mm. The plate 11 having wells of such a size is known as a microplate. The plan view shape of the well 13 may be a circle. When the plan view shape of the well 13 has a corner as in the square, the corner may be rounded.

The evaluation plate 10 is a plate in which after the evaluation target 14 corresponding to each well 13 of the plate 11 is injected and a certain culture treatment is performed, the toxicity of the evaluation target 14 (more specifically, a test substance) can be evaluated.

In the Ames test, the genotoxicity of the test substance and the cytotoxicity of the test substance are evaluated based on the evaluation target 14 in the evaluation plate 10 in a state in which evaluation can be performed. Evaluation of genotoxicity and cytotoxicity itself may be performed in accordance with the evaluation method in the Ames test (in particular, the miniaturized Ames test).

### (Evaluation of genotoxicity)

In the present embodiment, when the test substance has genotoxicity to the cells included in the evaluation target 14, the color of the indicator changes from purple to yellow as a positive result. Therefore, the genotoxicity of the test substance can be evaluated by determining the color (specifically, the color of the evaluation target 14 held in each well 13) of each well 13 of the evaluation plate 10. At this time, the colors of the wells 13 in the section Se1 (solvent control section or negative control section) and the wells 13 in the section Se8 (positive control section) may be used as a reference for evaluation.

In the present embodiment, when the test substance has genotoxicity to the cells included in the evaluation target 14, there is a possibility that colonies will be generated. That is, a case where colonies are detected corresponds to a positive result. Therefore, by counting the number of wells 13 having the above colonies, the genotoxicity of the test substance can be evaluated. At this time, the states (presence or absence of colonies) of the wells 13 in the section Se1 and the wells 13 in the section Se8 may be used as a reference for evaluation.

The states (such as the concentration of the test substance) of the evaluation targets 14 held in the plurality of wells 13 in each of the sections Se1 to Se8 are the same. Therefore, when the evaluation target 14 is evaluated, the colors (or states) of the plurality of wells 13 included in each of the sections Se1 to Se8 may be comprehensively determined.

### (Evaluation of cytotoxicity)

In the present embodiment, when the test substance has cytotoxicity to the cells included in the evaluation target 14, the transparency (specifically, the transparency of the evaluation target 14) of the well 13 changes due to the cell death or the like. Therefore, the cytotoxicity of the test substance can be evaluated based on the transparency of the well 13. At this time, the transparency of the wells 13 in the section Se1 (solvent control section or negative control section) may be used as a reference for evaluation. Even when the test substance is precipitated during culture, the transparency (specifically, the transparency of the evaluation target 14) of the well 13 changes. Therefore, the presence or absence of precipitation of the test substance can also be evaluated based on the transparency.

In the present embodiment, when the test substance has cytotoxicity to the cells included in the evaluation target 14, a foreign matter is generated as a result of the cell death. The foreign matter is a substance (or a structure) separated by an interface having a refractive index different from that of the periphery of the foreign matter. Therefore, the cytotoxicity of the test substance can be evaluated based on the presence or absence of a foreign matter in the well 13. At this time, the states of the wells 13 in the section Se1 (presence or absence of a foreign matter) may be used as a reference for evaluation.

Also in the cytotoxicity evaluation, the states (concentration of the test substance, and the like) of the evaluation targets 14 held in the plurality of wells 13 in each of the sections Se1 to Se8 are the same. Therefore, when the evaluation target 14 is evaluated, the transparency (or state) of the plurality of wells 13 included in each of the sections Se1 to Se8 may be comprehensively determined.

The determination of the color, the detection of colonies, the determination of the transparency, the detection of a foreign matter, and the like may be performed while the evaluator visually observes the evaluation plate 10, or may be performed by imaging the evaluation plate 10 and analyzing the obtained image. In the case of visual observation by the evaluator, the determination may also be performed by visually observing an image obtained by imaging the evaluation plate 10.

Next, a procedure of an example of the Ames test using the plate 11 will be specifically described.

### (Step 1A)

A tester (bacteria having amino acid auxotrophic mutation, for example, *S. typhimurium:* TA100 strain, TA98 strain, TA1535 strain, TA1537 strain, and *E. coli:* WP2 uvrA strain) to be used is inoculated into 60 mL of a nutrient broth medium and grown by shaking culture at 37°C for 9 to 10 hours.

### (Step 2A)

A required amount of a test substance (for example, a compound whose toxicity is to be evaluated) is weighed.

### (Step 3A)

The test substance prepared in the step 2A is dissolved or suspended in an appropriate medium, and this is serially diluted with the medium, to prepare a concentration series of the test substance solution. For example, when the same test substance is evaluated at six different concentrations, six test substance solutions having different concentrations are prepared.

### (Step 4A)

A solution of a positive control compound known to exhibit predetermined toxicity is prepared.

### (Step 5A)

The medium used for the preparation of the test substance solution, the concentration series of the test substance solution, and the solution of the positive control compound (collectively referred to as the test substance solution and the like) are placed in separate wells of a 24 well plate (plate having 24 wells) at 10 µL for each well.

### (Step 6A)

A culture solution (0.175 to 0.7 mL) of the tester (usually at a concentration exceeding 1 × 10⁹ cells/mL) and 6.83 to 6.3 mL of a culture medium (minimum culture medium to which a carbon source is added and in which amino acid is insufficient) are mixed. The mixture is referred to as a prepared bacterial solution under the condition of the absence of metabolic activation.

### (Step 7A)

To each well of the 24 well plate containing 10 µL of the test substance solution and the like in each well, 240 µL of the prepared bacterial solution under the condition of the absence of metabolic activation, which is prepared in the step 6A, is added.

### (Step 8A)

The 24 well plate containing the mixed solution of the step 7A is shaken at 37°C and 250 rpm for 90 minutes (pre-incubation) .

### (Step 9A)

After completion of the pre-incubation, 2.6 mL of an indicator medium (minimum culture medium containing a pH indicator and a carbon source, and lacking amino acid) is added to each well (250 µL) of the 24 well plate.

### (Step 10A)

The mixed solution in the step 9A is transferred at 50 µL each to 48 wells (1 section) included in each of the eight sections Se1 to Se8 of the plate 11 which is a 384 well plate so as to correspond to the sections Se1 to Se8. Similarly, a total of 3 or more 384 well plates (plates 11) are seeded.

### (Step 11A)

The 384 well plate (plate 11) is subjected to culturing at 37°C for 48 hours. As a result, the evaluation plate 10 is obtained.

### (Step 12A)

Thereafter, the change in color, presence or absence of colonies, presence or absence of a foreign matter, and change in transparency of each well 13 of the evaluation plate 10, which is the plate 11 in which the culture has been completed, are observed. Then, the number of wells having the change in color, colonies, and foreign matter is counted, and the section having the change in transparency is recorded.

### (Step 13A)

The presence or absence of the cytotoxicity caused by the test substance is determined by combining the presence or absence of a foreign matter and the result of the change in transparency.

### (Step 14A)

The presence or absence of genotoxicity is determined by combining the result of the change in color and the result of the presence or absence of colonies.

The order of the step 13A and the step 14A is not limited.

In a case where a test substance exhibiting toxicity after being metabolized by various enzymes in the living body is detected, in the step 6A, a mixed solution (S9 mixed solution) obtained by adding a coenzyme to an animal liver extract is prepared, and 0.175 to 0.7 mL of the culture solution of the tester, 5.78 to 5.25 mL of the culture medium, and 1.05 mL of the S9 mixed solution are mixed. The mixture is referred to as a prepared bacterial solution under the condition of the presence of metabolic activation. Thereafter, the steps after the step 7A are performed using the prepared bacterial solution under the condition of the presence of metabolic activation, instead of the prepared bacterial solution under the condition of the absence of metabolic activation.

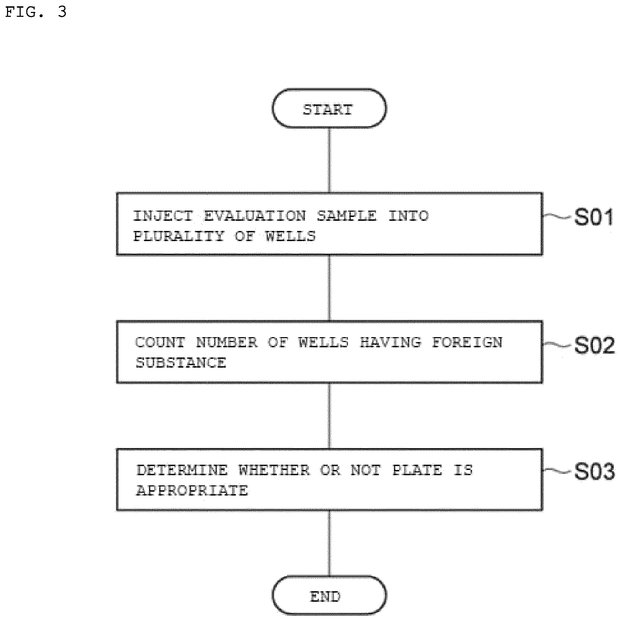

The inventors of the present application have found that the generated foreign matter may disappear depending on the material, the coating state, and the like of the plate 11. When the foreign matter disappears due to the influence of the plate 11 in this manner, toxicity evaluation cannot be properly performed. Therefore, next, a method for evaluating the appropriateness of the plate 11 will be described. Fig. 3 is a flowchart of an example of a method for evaluating a plate. The evaluation target 14 used for appropriateness evaluation of the plate 11 is referred to as an evaluation sample.

First, the evaluation sample is injected into the plurality of wells 13 of the plate 11 to be evaluated (injection step S01). The foreign matter is generated when cell death occures by the test substance. Therefore, in the method for evaluating a plate, an evaluation sample that includes cells and a test substance and that is prepared in advance so as to generate a foreign matter as a result of the cell death caused by the test substance is used. The number of wells 13 into which the evaluation sample is injected is, for example, 48 or more.

Examples of the cell used for the evaluation sample are bacteria. Examples of the bacteria used for the evaluation sample include *E. coli* (for example, *E. coli* WP2 uvrA strain) and *S. typhimurium* (for example, *S*. *typhimurium* TA100 strain). The test substance used for the evaluation sample is, for example, tert-butylhydroquinone, 1-decanol, 2-ethoxynaphthalene, 4-(1,1,3,3-tetramethylbutyl)phenol, diethyl chlorothiophosphate, or 4-chlorobenzyl chloride. For example, the test substance may be 1-decanol having a concentration of 62.5 ug/mL or more, or tert-butylhydroquinone having a concentration of 667 µg/mL or more.

Specific examples of the evaluation sample include the following first to fourth evaluation samples.

### (First evaluation sample)

The first evaluation sample is an evaluation sample using an *E. coli* WP2 uvrA strain as the cell, and 1-decanol having a concentration of 62.5 µg/mL as the test substance.

### (Second evaluation sample)

The second evaluation sample is an evaluation sample using an *E. coli* WP2 uvrA strain as the cell, and 1-decanol having a concentration of 125 µg/mL as the test substance.

### (Third evaluation sample)

The third evaluation sample is an evaluation sample using a *S. typhimurium* TA100 strain as the cell, and 1-decanol having a concentration of 667 ug/mL as the test substance.

### (Fourth evaluation sample)

The fourth evaluation sample is an evaluation sample using a *S. typhimurium* TA100 strain as the cell and 1-decanol having a concentration of 2,000 ug/mL as the test substance.

Next, the number of wells 13 having a foreign matter among the plurality of wells 13 is counted at the first counting timing at which the first predetermined time has elapsed after the evaluation sample is injected into the plurality of wells 13 (counting step S02). The counting of the wells 13 may be performed by directly visually observing the plate 11 into which the evaluation sample is injected. The counting may also be performed by acquiring an image of the plate 11 into which the evaluation sample is injected by an image capturing device, and counting the number of wells 13 having a foreign matter by visual observation or image analysis based on the image.

The first counting timing at which the first predetermined time has elapsed is a time at which the reliable presence of a foreign matter in the well can be ensured in a case where the plate is a plate that can retain the foreign matter. For example, when the presence or absence of a foreign matter, and the like are evaluated in the Ames test, the first counting timing may be a timing at which the evaluation is performed. The first counting timing may be after the test substance and the tester are cultured for 48 hours (corresponding to the first predetermined time) from injection of the test substance and the tester into the plate 11 as in the step 11A. The first counting timing is, for example, within 12 hours after the completion of culture for 48 hours from the injection of the evaluation sample into the plurality of wells 13, and is preferably within 3 hours.

In a case where the number of wells 13 having a foreign matter is 1 or more among the plurality of wells 13, the plate 11 is determined to be appropriate, and in a case where the number of wells 13 having a foreign matter is 0, the plate 11 is determined to be inappropriate (determination step S03).

An example of a method for evaluating the plate 11 will be specifically described.

### (Step 1B)

The same step as in the step 1A is performed except that the cells used for the evaluation sample of the plate 11 are used as the tester to be used. Specifically, the cells for the evaluation sample are inoculated into 60 mL of a nutrient broth medium and grown by shaking culture at 37°C for 9 to 10 hours.

### (Step 2B)

The same step as in the step 2A is performed except that the test substance used for the evaluation sample of the plate 11 is used as the test substance to be used. Specifically, a required amount of the test substance for the evaluation sample is weighed.

### (Step 3B)

The test substance in the step 2B is dissolved or suspended in an appropriate medium, and a test substance solution is prepared so that the concentration of the test substance is 25 times the concentration that affects the survival of cells.

### (Step 4B)

The test substance solution obtained in the step 3B is placed at 10 µL each in the well of a 24 well plate.

### (Step 5B)

A prepared bacterial solution under the condition of the absence of metabolic activation is prepared in the same manner as in the step 6A. Specifically, 0.175 to 0.7 mL of a cell culture solution (usually at a concentration exceeding 1 × 10⁹ cells/mL) and 6.83 to 6.3 mL of a culture medium (minimum culture medium to which a carbon source is added and in which amino acid is insufficient) are mixed to prepare a prepared bacterial solution under the condition of the absence of metabolic activation.

### (Step 6B)

To each well of the 24 well plate containing 10 µL of the test substance solution and the like in each well, 240 µL of the prepared bacterial solution under the condition of the absence of metabolic activation, which is prepared in the step 5B, is added.

### (Step 7B)

The 24 well plate containing the mixed solution prepared in the step 6B is shaken at 37°C and 250 rpm for 90 minutes (pre-incubation).

### (Step 8B)

After completion of the pre-incubation, 2.6 mL of an indicator medium (minimum culture medium containing a pH indicator and a carbon source, and lacking amino acid) is added to each well (250 µL) of the 24 well plate.

### (Step 9B)

The mixed solution obtained in the step 8B is transferred at 50 µL each to 48 wells (1 section) of a 384 well plate (plate 11).

### (Step 10B)

The 384 well plate (plate 11) is subjected to culturing at 37°C for 48 hours.

### (Step 11B)

In the plate 11 in which the culture has been completed, the presence or absence of a foreign matter among 48 wells 13 into which the mixed solution has been injected in the step 9B is observed, and the number of wells 13 including a foreign matter is counted. This step corresponds to the counting step S02.

### (Step 12B)

Thereafter, as the step 12B, the determination step S03 is performed.

The plate 11 that is evaluated as being appropriate when performing the method for evaluating a plate can retain a foreign matter. That is, such a plate is a plate in which a foreign matter does not disappear due to the influence of the plate 11. Therefore, when cytotoxicity is evaluated according to the evaluation method in the Ames test described above using the plate 11 evaluated as being appropriate by the method for evaluating a plate, evaluation of the toxicity of the test substance to cells of can be accurately performed.

When the plate 11 is determined to be appropriate in the determination step S03, the number of wells 13 having a foreign matter among the plurality of wells 13 is counted again at the second counting timing at which the second predetermined time has elapsed from the first counting timing, and then the determination step S03 may be performed again based on the counting result at the second counting timing. Therefore, for example, the method for evaluating a plate may further include the following steps.

After the steps 1B to 12B are performed, the plate 11 is stored in a refrigerator (for example, 4°C). One day after the start of storage (corresponding to the second counting timing), the presence or absence of a foreign matter in each well is observed, the number of wells having a foreign matter is counted, and the determination step S03 is performed based on the result.

Here, one day after the start of storage has been exemplified as the second counting timing, but two days, three days, four days, or five days after the start of storage may be used. The counting of the number of wells having a foreign matter and the determination step S03 based on the result may be performed after one day, two days, three days, four days, and five days, respectively.

In the toxicity evaluation test such as the Ames test, there may be a case where the presence or absence of a foreign matter is not determined at the first counting timing, but determined, for example, at the second counting timing at which a predetermined time has elapsed while the plate 11 is stored at a constant temperature (for example, 10°C or lower). As described above, by counting again the number of wells 13 having a foreign matter among the plurality of wells 13 at the second counting timing and performing the determination step S03 again, it is possible to evaluate whether or not the plate 11 is an appropriate plate even for a toxicity evaluation test of determining the presence or absence of a foreign matter at the second counting timing. Therefore, the second counting timing may be any timing as long as the presence or absence of a foreign matter is evaluated after the first counting timing in the toxicity evaluation test to be performed. Although 10°C or lower is exemplified as the constant temperature, the constant temperature may be, for example, 0°C or higher and 10°C or lower.

In an embodiment in which the evaluation sample is injected into at least 48 wells 13 and the number of wells 13 including a foreign matter is counted, the number of wells 13 used for determination of the presence or absence of a foreign matter can be ensured, so that the plate 11 can be more accurately evaluated.

Various embodiments of the present invention have been described above. However, the present invention is not limited to the illustrated embodiments, and is intended to encompass the scope indicated by the claims and to include all modifications within the meaning and scope equivalent to the claims.

The foreign matter is a substance (or structure) that is generated when cell death occures, for example, a substance (or structure) that was not originally contained, and can be, for example, a substance whose boundary between the contour and the inside thereof is clear. For example, the foreign matter is a foreign matter is a substance (or a structure) separated by an interface having a refractive index different from that of the periphery of the foreign matter. The foreign matter is, for example, oil masses, bubbles, and the like.

In the above description, the evaluation test of cytotoxicity according to the procedure of the Ames test has been described as an example. However, the plate determined to be appropriate by the method for evaluating a plate can be applied to a test for evaluating cytotoxicity based on the presence or absence of a foreign matter generated as a result of the cell death due to the influence of the test substance.

The number of wells 13 into which the evaluation sample is injected may be 12 or more and less than 48, or may be, for example, 48 or more and 384 or less as long as the counting result of the wells 13 having a foreign matter is a number that is statistically meaningful or meaningful in the toxicity evaluation test using a plate (for example, the number of wells constituting one section in the plate 11) .

The evaluation sample used for evaluation of a plate may be a sample in which the cells and the test substance are selected and adjusted so that a foreign matter is generated, using a plate that has been found to reliably retain a foreign matter (hereinafter, referred to as "foreign matter retaining plate"). For example, the evaluation sample used for evaluation of a plate may be a sample in which the cells and the test substance are adjusted so that at least one well has a foreign matter when the number of wells having a foreign matter among the plurality of wells of the foreign matter retaining plate is counted at the first counting timing at which the first predetermined time has elapsed since the evaluation sample is injected into the plurality of wells of the foreign matter retaining plate in the method for evaluating a plate. In the case of adjusting the evaluation sample using the foreign matter retaining plate, an example of the number of the plurality of wells into which the evaluation sample is injected in the foreign matter retaining plate may be the same as the case of injecting the evaluation sample into a plate to be evaluated in the method for evaluating a plate. The evaluation sample used for evaluation of a plate may be a sample that is known to generate a foreign matter in the Ames test or the like.

The exemplified embodiments and modifications may be appropriately combined without departing from the gist of the present invention.

### EXAMPLES

Next, examples will be described. The present invention is not limited to the following examples.

In the examples, the following six plates A to H were evaluated. The plates A to H had the same configuration except for the material and the coating state shown below, and had 384 wells similarly to the plate 11 illustrated in Fig. 1.

### (Plate A)

The material of the plate A was polystyrene. The surface (including the inner surface and the bottom surface of the well 13) of the plate A was subjected to tissue culture (TC) treatment.

### (Plate B)

The material of the plate B was polystyrene. The plate B was not subjected to coating.

### (Plate C)

The plate C had a glass plate, and was formed by bonding a structure having 384 wells 13 onto the glass plate. The material of the structure was a cyclic olefin copolymer (COC). Therefore, the material of the bottom surface of each well 13 of the plate C was glass, and the material of the wall surface was COC. In the plate C, the bottom surface of the well 13 was coated with fibronectin.

### (Plate D)

The material of the plate D was polystyrene. The surface of the plate D was coated with Poly-D-Lysine.

### (Plate E)

The plate E had a glass plate, and was formed by bonding a structure having 384 wells 13 onto the glass plate. The material of the structure was COC. Therefore, the material of the bottom surface of each well 13 of the plate E was glass, and the material of the wall surface was COC. The plate E was not subjected to surface coating.

### (Plate F)

The material of the plate F was a cycloolefin polymer. The plate F was not subjected to surface coating. That is, the plate F was a cycloolefin polymer plate that was not subjected to surface coating.

### (Plate G)

The plate G had a glass plate, and was formed by bonding a structure having 384 wells 13 onto the glass plate. The material of the structure was polystyrene. Therefore, the material of the bottom surface of each well 13 of the plate G was glass, and the material of the wall surface was polystyrene. The plate G was not subjected to surface coating.

### (Plate H)

The material of the plate H was COC. The surface of the plate H was subjected to tissue culture (TC) treatment.

The cells used in Examples 1 to 6 were those available from the National Institute of Health Sciences, the National Institute of Technology and Evaluation, Biotechnology Center, American Type Culture Collection, Ames Test Kit ("Ames MPF (trade mark) Penta I Microplate Format Mutagenicity Assay") manufactured by Xenometrix, and the like. The test substances used in Examples 1 to 6 were manufactured by Tokyo Chemical Industry Co., Ltd.

### (Example 1)

In Example 1, the steps 1B to 8B were performed using the following as the cells and the test substance for the evaluation sample. Thereafter, in the step 9B, the mixed solution prepared in the step 8B was transferred to the plate A, the plate B, and the plate C as described in the step 9B, and then the steps 10B to 12B were performed.

### <Cells and test substance for evaluation sample of Example 1>

Cells: *E. coli* WP2 uvrA strain
Test substance: 1-decanol

The medium used in the step 3B of Example 1 was DMSO. In the step 5B, 0.7 mL of cells were mixed into 6.3 mL of a culture medium. The concentration in the step 6B was 62.5 µg/mL.

In Example 1, the presence or absence of a foreign matter in each well 13 was visually observed and the number of wells including a foreign matter was counted in the step 11B, the plate 11 was then stored at 4°C, and the presence or absence of a foreign matter in each well 13 was visually observed and the number of wells including a foreign matter was counted after one day, two days, three days, four days, and five days, respectively.

The results of Example 1 were as shown in Table 1. In Table 1, the number of wells in which a foreign matter could be confirmed among 48 wells 13 is shown for each counting timing. The phrase "immediately after completion of culture" in Table 1 means that a foreign matter was visually observed at the time point when 48 hours of culture was completed.

**[Table 1]**

| Plate | Immediately after completion of culture | After 1 day | After 2 days | After 3 days | After 4 days | After 5 days |
|---|---|---|---|---|---|---|
| Plate A | 0 | 0 | 0 | 0 | 0 | 0 |
| Plate B | 11 | 0 | 0 | 0 | 0 | 0 |
| Plate C | 18 | 17 | 17 | 17 | 17 | 17 |

### (Example 2)

In Example 2, the steps 1B to 8B were performed using the following as the cells and the test substance for the evaluation sample. Thereafter, in the step 9B, the mixed solution prepared in the step 8B was transferred to the plate A, the plate B, and the plate C as described in the step 9B, and then the steps 10B to 12B were performed.

### <Cells and test substance for evaluation sample of Example 2>

Cells: *E. coli* WP2 uvrA strain
Test substance: 1-decanol

The medium used in the step 3B of Example 2 was DMSO. In the step 5B, 0.7 mL of cells were mixed into 6.3 mL of a culture medium. The concentration in the step 6B was 125 µg/mL.

In Example 2, as in Example 1, the presence or absence of a foreign matter in each well 13 was visually observed and the number of wells including a foreign matter was counted in the step 11B, the plate 11 was then stored at 4°C, and the presence or absence of a foreign matter in each well 13 was visually observed and the number of wells including a foreign matter was counted after one day, two days, three days, four days, and five days, respectively. The results of Example 2 were as shown in Table 2. The meaning of "immediately after completion of culture" in Table 2 was the same as that in the case of Table 1.

**[Table 2]**

| Plate | Immediately after completion of culture | After 1 day | After 2 days | After 3 days | After 4 days | After 5 days |
|---|---|---|---|---|---|---|
| Plate A | 0 | 0 | 0 | 0 | 0 | 0 |
| Plate B | 15 | 0 | 0 | 0 | 0 | 0 |
| Plate C | 18 | 18 | 18 | 18 | 18 | 18 |

### (Example 3)

In Example 3, the steps 1B to 8B were performed using the following as the cells and the test substance for the evaluation sample. Thereafter, in the step 9B, the mixed solution prepared in the step 8B was transferred to the plate A, the plate B, and the plate C as described in the step 9B, and then the steps 10B to 12B were performed.

### <Cells and test substance for evaluation sample of Example 3>

Cells: *S. typhimurium* TA100 strain
Test substance: 1-decanol

The medium used in the step 3B of Example 3 was DMSO. In the step 5B, 0.35 mL of cells were mixed into 6.65 mL of a culture medium. The concentration in the step 6B was 667 µg/mL.

In Example 3, as in Example 1, the presence or absence of a foreign matter in each well 13 was visually observed and the number of wells including a foreign matter was counted in the step 11B, the plate 11 was then stored at 4°C, and the presence or absence of a foreign matter in each well 13 was visually observed and the number of wells including a foreign matter was counted after one day, two days, three days, four days, and five days, respectively. The results of Example 3 were as shown in Table 3. The meaning of "immediately after completion of culture" in Table 3 was the same as that in the case of Table 1.

**[Table 3]**

| Plate | Immediately after completion of culture | After 1 day | After 2 days | After 3 days | After 4 days | After 5 days |
|---|---|---|---|---|---|---|
| Plate A | 0 | 0 | 0 | 0 | 0 | 0 |
| Plate B | 21 | 0 | 0 | 0 | 0 | 0 |
| Plate C | 23 | 23 | 23 | 23 | 23 | 23 |

### (Example 4)

In Example 4, the steps 1B to 8B were performed using the following as the cells and the test substance for the evaluation sample. Thereafter, in the step 9B, the mixed solution prepared in the step 8B was transferred to the plate A, the plate B, and the plate C as described in the step 9B, and then the steps 10B to 12B were performed.

### <Cells and test substance for evaluation sample of Example 4>

Cells: *S. typhimurium* TA100 strain
Test substance: 1-decanol

The medium used in the step 3B of Example 4 was DMSO. In the step 5B, 0.35 mL of cells were mixed into 6.65 mL of a culture medium. The concentration in the step 6B was 2,000 µg/mL.

In Example 4, as in Example 1, the presence or absence of a foreign matter in each well 13 was visually observed and the number of wells including a foreign matter was counted in the step 11B, the plate 11 was then stored at 4°C, and the presence or absence of a foreign matter in each well 13 was visually observed and the number of wells including a foreign matter was counted after one day, two days, three days, four days, and five days, respectively.

The results of Example 4 were as shown in Table 4. In Table 4, the number of wells in which a foreign matter could be confirmed among 48 wells 13 is shown for each counting timing. The meaning of "immediately after completion of culture" in Table 4 was the same as that in the case of Table 1.

**[Table 4]**

| Plate | Immediately after completion of culture | After 1 day | After 2 days | After 3 days | After 4 days | After 5 days |
|---|---|---|---|---|---|---|
| Plate A | 0 | 0 | 0 | 0 | 0 | 0 |
| Plate B | 27 | 0 | 0 | 0 | 0 | 0 |
| Plate C | 24 | 24 | 24 | 24 | 24 | 24 |

### (Example 5)

In Example 5, the steps 1B to 8B were performed using the following as the cells and the test substance for the evaluation sample. Thereafter, in the step 9B, the mixed solution prepared in the step 8B was transferred to the plate A, the plate D, and the plate E as described in the step 9B, and then the steps 10B to 12B were performed.

### <Cells and test substance for evaluation sample of Example 5>

Cells: *S. typhimurium* TA98 strain
Test substance: tert-butylhydroquinone

The medium used in the step 3B of Experimental Example 5 was DMSO. In the step 5B, 0.7 mL of cells were mixed into 6.3 mL of a culture medium. The concentration in the step 6B was 2,500 µg/mL.

In Example 5, the presence or absence of a foreign matter in each well 13 was visually observed, and the number of wells including a foreign matter was counted in the step 11B. The results of Example 5 were as shown in Table 5. In Table 5, the number of wells in which a foreign matter could be confirmed among 48 wells 13 is shown. The meaning of "immediately after completion of culture" in Table 5 was the same as that in the case of Table 1.

**[Table 5]**

| Plate | Immediately after completion of culture |
|---|---|
| Plate A | 0 |
| Plate D | 48 |
| Plate E | 42 |

### (Example 6)

In Example 6, the steps 1B to 8B were performed using the following as the cells and the test substance for the evaluation sample. Thereafter, in the step 9B, the mixed solution prepared in the step 8B was transferred to the plate A, the plate F, the plate G, and the plate H as described in the step 9B, and then the steps 10B to 12B were performed.

### <Cells and test substance for evaluation sample of Example 6>

Cells: *E. coli* WP2 uvrA strain
Test substance: 1-decanol

The medium used in the step 3B of Example 6 was DMSO. In the step 5B, 0.7 mL of cells were mixed into 6.3 mL of a culture medium. The concentration in the step 6B was 125 µg/mL.

In Example 6, the presence or absence of a foreign matter in each well 13 was visually observed, and the number of wells including a foreign matter was counted in the step 11B. The results of Example 6 were as shown in Table 6. In Table 6, the number of wells in which a foreign matter could be confirmed among 48 wells 13 is shown. The meaning of "immediately after completion of culture" in Table 6 was the same as that in the case of Table 1.

**[Table 6]**

| Plate | Immediately after completion of culture |
|---|---|
| Plate A | 0 |
| Plate F | 5 |
| Plate G | 18 |
| Plate H | 48 |

### (Evaluation of examples)

In Examples 1 to 6, when counting is performed immediately after completion of culture, wells retaining a foreign matter are present in the plate B and the plate C. Therefore, it can be found that the combination of the cells and the test substance and the concentration of the test substance used in Examples 1 to 6 are conditions of generating a foreign matter, and the plate B, the plate C, the plate D, the plate E, the plate F, the plate G, and the plate H are plates that can retain a foreign matter immediately after completion of culture. On the other hand, in the plate A, the well 13 retaining a foreign matter was not present immediately after completion of culture in Examples 1 to 6. Therefore, the plate A is a plate that cannot retain a foreign matter.

Therefore, in the cytotoxicity evaluation test for determining the presence or absence of a foreign matter immediately after culture, the plates B to H are evaluated as being appropriate, that is, plates that can properly evaluate the presence or absence of a foreign matter, whereas the plate A can be evaluated as a plate that cannot properly evaluate the presence or absence of a foreign matter.

From the counting results after one day, two days, three days, four days, and five days in Examples 1 to 4, the plate C is evaluated as being appropriate, that is a plate that can properly evaluate the presence or absence of a foreign matter for the cytotoxicity evaluation test for determining the presence or absence of a foreign matter after one day, two days, three days, four days, and five days, respectively, similarly to the case immediately after culture. On the other hand, in the plate A and the plate B, the wells 13 retaining a foreign matter were present immediately after culture, but the wells 13 retaining a foreign matter were not present after one day. Therefore, when the presence or absence of a foreign matter is evaluated after one day, the plate A and the plate B can be evaluated as plates that cannot properly evaluate the presence or absence of a foreign matter.

In the results immediately after culture, the same results were obtained in Examples 1 to 6. Therefore, for example, when the evaluation sample of Example 1 (cells, test substance, and test substance concentration used in Example 1) is used as a reference sample for plate evaluation, it can be found that the plate can be appropriately evaluated by using the reference sample even in the case of a combination of another cell, test substance, and test substance concentration as in Examples 2 to 6.

Similarly, when the evaluation sample of Example 2 (cells, test substance, and test substance concentration used in Example 2) is used as a reference sample for plate evaluation, it can be found that the plate can be appropriately evaluated by using the reference sample even in the case of a combination of another cell, test substance, and test substance concentration as in Example 1 and Examples 3 to 6. The same applies to the case where the evaluation samples of Examples 3 to 6 are used as reference samples for plate evaluation.

Therefore, it was verified that the evaluation samples used in Examples 1 to 6 can be used as an evaluation sample for evaluating a plate immediately after culture.

The same applies to the case where the counting timing is after one day, two days, three days, four days, and five days. That is, it was verified that the evaluation samples used in Examples 1 to 4 can be used as an evaluation sample for evaluating a plate when each of one day, two days, three days, four days, and five days is the counting timing.

As described above, for example, the plate C is a foreign matter retaining plate that can retain a foreign matter. Although the plate C had 384 wells, the number of the plurality of wells of the foreign matter retaining plate is not limited to 384. Therefore, the foreign matter retaining plate may be a plate including: a glass plate; and a resin layer disposed on a surface of the glass plate and formed of a cyclic olefin copolymer, in which a plurality of through holes to be a plurality of wells of the foreign matter retaining plate are formed in the resin layer, and a region exposed through the plurality of through holes in the surface is coated with fibronectin. In this case, as described above, the evaluation sample may be a sample in which the cells and the test substance are adjusted so that at least one well has a foreign matter when the number of wells having a foreign matter among the plurality of wells of the foreign matter retaining plate is counted at the first counting timing at which the first predetermined time described in the method for evaluating a plate has elapsed since the evaluation sample is injected into the plurality of wells of the foreign matter retaining plate.

### DESCRIPTION OF REFERENCE SIGNS

- 10: Evaluation plate
- 11: Plate
- 13: Well
- 14, 14a to 14h: Evaluation target

## Claims

1. A method for evaluating a plate including a plurality of wells, the method comprising:
an injection step of injecting an evaluation sample including a cell and a test substance into the plurality of wells, the evaluation sample being adjusted in advance so that a foreign matter is generated as a result of cell death caused by the test substance;
a counting step of counting the number of wells having the foreign matter among the plurality of wells at a first counting timing at which a first predetermined time has elapsed since the evaluation sample is injected into the plurality of wells; and
a determination step of determining that the plate is appropriate when the number of wells having the foreign matter among the plurality of wells is one or more, and determining that the plate is inappropriate when the number of wells having the foreign matter is zero.

2. The method for evaluating a plate according to claim 1, wherein the first counting timing is after the evaluation sample is cultured for 48 hours from injection of the evaluation sample into the plurality of wells.

3. The method for evaluating a plate according to claim 1 or 2, wherein the plurality of wells has 48 or more wells.

4. The method for evaluating a plate according to claim 1 or 2, wherein the determination step is performed again after the number of wells having the foreign matter among the plurality of wells is counted at a second counting timing at which a second predetermined time has elapsed while the plate is stored at a constant temperature from the first counting timing.

5. The method for evaluating a plate according to claim 4, wherein the constant temperature is 10°C or lower.

6. The evaluation method according to any one of claims 1 to 5, wherein the cell is a bacterium.

7. The method for evaluating a plate according to any one of claims 1 to 6, wherein the cell is *E. coli* or *S. typhimurium.*

8. The method for evaluating a plate according to any one of claims 1 to 7, wherein
the evaluation sample is a sample in which a cell and a test substance are adjusted so that at least one well has the foreign matter when the number of wells having the foreign matter among a plurality of wells of a foreign matter retaining plate is counted at the first counting timing at which the first predetermined time has elapsed since the evaluation sample is injected into the plurality of wells of the foreign matter retaining plate, and
the foreign matter retaining plate is a plate including: a glass plate; and a resin layer that is disposed on a surface of the glass plate and is formed of a cyclic olefin copolymer, wherein a plurality of through holes to be the plurality of wells of the foreign matter retaining plate are formed in the resin layer, and a region exposed through the plurality of through holes in the surface is coated with fibronectin.

9. The method for evaluating a plate according to any one of claims 1 to 8, wherein the test substance is tert-butylhydroquinone, 1-decanol, 2-ethoxynaphthalene, 4-(1,1,3,3-tetramethylbutyl)phenol, diethyl chlorothiophosphate, or 4-chlorobenzyl chloride.

10. The method for evaluating a plate according to any one of claims 1 to 9, wherein the test substance is 1-decanol having a concentration of 62.5 ug/mL or more or tert-butylhydroquinone having a concentration of 667 ug/mL or more.

11. The method for evaluating a plate according to any one of claims 1 to 10, wherein the evaluation sample is an evaluation sample using an *E. coli* WP2 uvrA strain as the cell and 1-decanol having a concentration of 62.5 ug/mL or 125 ug/mL as the test substance, or an evaluation sample using a *S. typhimurium* TA100 strain as the cell and 1-decanol having a concentration of 667 ug/mL or 2,000 ug/mL as the test substance.

12. A method for evaluating toxicity of a test substance, the method comprising a step of injecting an evaluation target including: a tester including a cell; and a test substance into a plurality of wells of the plate determined to be appropriate by the method for evaluating a plate according to any one of claims 1 to 11, and evaluating toxicity of the test substance.
